# EUROPEAN PATENT SPECIFICATION

(11) **EP 4 699 562 B1**
(45) Date of publication and mention of the grant of the patent: **07.10.2026**
(21) Application number: 24203358.7
(22) Date of filing: 27.09.2024
(51) Int. Cl.: A61B 34/20

(54) **POSITION TRACKING APPARATUS FOR HEAD AND NECK IGS USING MAGNETIC FIELD**
POSITIONSVERFOLGUNGSVORRICHTUNG FÜR KOPF-HALS-IGS UNTER VERWENDUNG EINES MAGNETFELDES
APPAREIL DE SUIVI DE POSITION POUR DES IG DE TÊTE ET DE COU UTILISANT UN CHAMP MAGNÉTIQUE

(30) Priority: 23.08.2024 KR 20240113635
(43) Date of publication of application: 25.02.2026
(73) Proprietor: Mega Medical Co., Ltd, Wonju-si Gangwon-do 26365 (KR)
(72) Inventor: KIM, Byungjang, 21004 Incheon (KR); LIM, Kyungsoon, 24401 Chuncheon-si (KR); JANG, Jiwoon, 34875 Daejeon (KR); PARK, Jisu, 26352 Wonju-si (KR); PARK, Junsu, 26352 Wonju-si (KR)
(74) Representative: Schiweck Weinzierl Koch Patentanwälte Partnerschaft mbB

(56) References cited:
- EP-A1- 4 074 275
- WO-A1-2023/144668
- US-A1- 2017 027 541
- US-A1- 2020 107 889
- US-A1- 2023 270 505

## Description

### CROSS-REFERENCE TO RELATED APPLICATIONS

This application claims the priority of Korean Patent Application No. 10-2024-0113635 filed on August 23, 2024, in the Korean Intellectual Property Office.

### BACKGROUND

### Field

The present disclosure relates to a position tracking apparatus for head and neck image guided surgery (IGS) using a magnetic field, and more particularly, to a position tracking apparatus for head and neck IGS using a magnetic field that is used for head and neck IGS (otolaryngology, ophthalmology, dentistry, plastic surgery, etc.), generates an alternating (AC) magnetic field around a patient while a doctor is performing surgery and then receives a detection result signal based on an induced current generated in response to the AC magnetic field to register a position of a surgical instrument in an affected area and output a structure and direction of a surgical area in a form that can be seen with the naked eye by a medical practitioner.

### Description of the Related Art

In general, patients with malignant tumors, cerebral thrombosis, Parkinson's disease, epilepsy, hyperkinesia, and external nervous system diseases are treated with medication or physical therapy in the early stages. Thereafter, when it is determined that treatment is no longer possible, surgical operation is performed.

At this time, in the case of conventional surgery, to identify a position of the affected area and a mutual position between a patient and a surgical instrument, a pillar is fixed to a patient's bone, a position tracking marker is installed on the pillar, the position tracking marker is also installed on the surgical instrument, a camera is installed to track the marker, and a computer and monitor are installed to guide the surgery based on a video captured by the camera.

However, today's surgeons may only rely on preoperative data before performing brain surgery. However, the position data of the surgical site may often become inaccurate due to small movements of a brain or inappropriate manipulation of a stereotactic positioning system during surgery, which causes unsatisfactory surgical results or postoperative side effects. US 2023/270505 A1 discloses an electromagnetic tracking apparatus according to the introductory statement of claim 1.

### SUMMARY

The invention is defined by claim 1. An object to be achieved by the present disclosure is to provide a position tracking apparatus for head and neck image guided surgery (IGS) using a magnetic field that is used for head and neck IGS (otolaryngology, ophthalmology, dentistry, plastic surgery, etc.), and generates an alternating (AC) magnetic field around a patient while a doctor is performing surgery and then receives a detection result signal based on an induced current generated in response to the AC magnetic field to register a position of a surgical instrument in an affected area and output a structure and direction of a surgical area in a form that can be seen with the naked eye by a medical practitioner.

The term "head and neck" is throughout the application used in the normal meaning of "head and/or neck" as used in the field of the invention. Thus, e.g. the IGS only of the head of a patient is included in the term head and neck IGS,

According to an aspect of the present disclosure, there is provided a position tracking apparatus 100 for head and neck IGS using a magnetic field, including: a main body part 110; a field generator 120 that is connected to the main body part 110 and generates externally an alternating (AC) magnetic field around an affected area through an internal coil according to a control signal transmitted from the main body part 110; an electromagnetic (EM) patient tracker 130 that has one side worn on a patient's head and neck in IGS while the other side is connected to the main body part 110 and detects the AC magnetic field and transmits a detection result signal to the main body part 110; an EM pointer 140 that has one side inserted into the patient's head and neck in IGS while the other side is connected to the main body part 110 and generates an induced current in response to the AC magnetic field to notify a position within the affected area; a moving cart 150 that has the main body part 110 and the field generator 120 mounted thereon and is configured to be movable; an EM instrument tracker 160 that is configured such that one side is connected to the main body part 110 and the other side is connected to a commercial surgical instrument, and guides the commercial surgical instrument inserted into the patient's head and neck in IGS to generate the induced current in response to the AC magnetic field; a forceps clamp 170 that is fixed to the moving cart 150 and has one side connected to surgical forceps to fix the forceps; an all-round clamp 180 that is fixed to the moving cart 150 and clamps and fixes the commercial surgical instrument; and a storage moving cart 190 that is provided with a storage space for receiving the EM patient tracker 130, the EM pointer 140, the EM instrument tracker 160, the forceps clamp 170, and the all-round clamp 180, and is configured to be movable, in which as the EM pointer 140 generates the induced current in response to the AC magnetic field generated externally by the field generator 120, when the EM patient tracker 130 worn on the patient's head and neck in IGS transmits the detection result signal based on the induced current, the main body part 110 calculates a distance, position, and direction of the EM pointer 140 within the patient's head and neck in IGS and maps an image generated by calculating the distance, position, and direction of the EM pointer 140 to a previously captured facial X-ray image of the patient and outputs the mapped image through a display device connected to the main body part 110.

A front portion of the main body part 110 may be provided with: a power switch 111; at least two USB 3.0 connection ports 112 into which a USB memory device is inserted; a connection port 113 for a field generator into which a cable terminal of the field generator 120 is inserted; a connection port 114 for an EM patient tracker into which a cable terminal of the EM patient tracker 130 is inserted; a connection port 115 for an EM pointer into which a cable terminal of the EM pointer 140 is inserted; and a connection port 116 for an EM tube pointer into which a cable terminal of the EM tube pointer is inserted, and a rear portion of the main body part 110 may be provided with: a power grid 117; a grounding potential equalization terminal 118; and an additional connection port 119 that includes a USB 2.0 connection port, a DVI connection port, and an HDMI connection port.

The main body part 110 may amplify the detection result signal transmitted from the EM patient tracker 130 using an Intermediate stage interface unit (ISIU) amplification module, may preprocess, in an SCU, the detection result signal amplified by the ISIU amplification module and then may process the preprocessed detection result signal into three-dimensional position information, and may transmit the processed signal to a video output module to be output through the display device.

When the power switch 111 is operated in an on state by a medical practitioner, the main body part 110 may execute a dedicated application for position tracking for the IGS, retrieve image data for the corresponding patient based on unique ID information for each patient, and then output a DICOM image to the display device.

The main body part 110 may retrieve the image data for the corresponding patient, and then output a notification to the medical practitioner through the display device to recheck whether the unique ID information and information on a patient to be operated match.

A fixed support hole 121 may be formed on each side and rear surface of the field generator 120 with which a fixed support for mounting on the moving cart 150 or a surgical bed is fastened, and the field generator 120 may receive power for generating the AC magnetic field from the main body part 110.

A medical adhesive pad is disposed on a lower side surface of the EM patient tracker 130, and the EM patient tracker 130 may be attached to a patient's forehead through adhesive strength of the medical adhesive pad.

When the EM patient tracker 130 is attached to the patient's forehead through the medical adhesive pad, the main body part 110 may output a screen for selecting one of a landmark type or a surface type through the display device to measure a registration value.

When the landmark type is selected by the medical practitioner, the main body part 110 may output a guide screen for designating reference points on the display screen, and guide the medical practitioner to move the position of the EM pointer 140 through the guide screen so that the designated point positions are sequentially contacted.

When the surface type is selected by the medical practitioner, the main body part 110 may output a guide screen for designating starting points on the display screen, and guide the medical practitioner to move the position of the EM pointer 140 through the guide screen so that the EM pointer 140 comes into contact with a patient's face surface along a preset path starting from the starting point.

The main body part 110 may guide a process of guiding the position movement of the EM pointer 140 to be repeated until the registration value reaches a preset registration value.

In the process of guiding the medical practitioner to move the position of the EM pointer 140 through the guide screen so that the EM pointer 140 is contacted along the preset path starting from the starting point on the patient's face surface, the main body part 110 may designate an area between the patient's forehead and eyebrows as the starting point, and then guide a first path that connects left eye area and right eye area of the patient in a figure-eight shape, a second path that crosses a patient's nose and leads to a philtrum, and a third path that starts from the philtrum and is raised to a glabella while zigzagging across a bridge of the nose through the guide screen.

When the EM pointer 140 is positioned within an effective range of the AC magnetic field generated externally through the field generator 120 within the patient's head and neck in IGS, the main body part 110 may display a green icon on the screen of the display device, and when the EM pointer 140 is determined to be out of the effective range of the AC magnetic field within the patient's head and neck in IGS, the main body part 110 may display a red icon and output a warning notification screen to notify that the EM pointer 140 is out of the effective range on the screen of the display.

The all-round clamp 180 may include: a small all-round clamp 181 that clamps the commercial surgical instrument having a diameter of 2 mm to 6 mm and fixes the commercial surgical instrument to the moving cart 150; a medium all-round clamp 182 that clamps the commercial surgical instrument having a diameter of 6 mm to 10 mm and fixes the commercial surgical instrument to the moving cart 150; and a large all-round clamp 183 that clamps the commercial surgical instrument having a diameter of 10 mm to 16 mm and fixes the commercial surgical instrument to the moving cart 150.

The position tracking apparatus may further include: an additional EM instrument tracker 200 that is configured such that one side is connected to the main body part 110 and the other side is connected to a commercial balloon catheter, and guides the commercial balloon catheter inserted into the patient's head and neck in IGS to generate the induced current in response to the AC magnetic field; an EM tube pointer 300 that has one side inserted into the patient's head and neck in IGS while the other side is connected to the main body part 110, generates the induced current in response to the AC magnetic field to notify a position within the head and neck in IGS, and sucks foreign substances within the head and neck in IGS through a suction hole formed at a center; and an EM endoscope shaft 400 that has one side inserted into the patient's head and neck in IGS while the other side is coupled to a commercial endoscope, and generates the induced current in response to the AC magnetic field to notify a position of the commercial endoscope within the head and neck in IGS.

According to an embodiment of the present disclosure, by generating an alternating (AC) magnetic field around a patient while a doctor is performing surgery and then receiving a detection result signal based on an induced current generated in response to the AC magnetic field, it is possible to register a position of a surgical instrument in an affected area and output a structure and direction of a surgical area in a form that can be seen with the naked eye by a medical practitioner.

In particular, according to an embodiment of the present disclosure, by mapping an image of an EM pointer whose geometric structure is registered within an X-ray image of a patient's face taken before surgery and outputting the mapped image through a video output device, it is possible to more accurately identify a position of a surgical instrument within an affected area.

### BRIEF DESCRIPTION OF THE DRAWINGS

The above and other aspects, features and other advantages of the present disclosure will be more clearly understood from the following detailed description taken in conjunction with the accompanying drawings, in which:
FIG. 1 is a diagram illustrating a configuration of a position tracking apparatus 100 for head and neck IGS using a magnetic field according to an embodiment of the present disclosure;
FIG. 2 is a diagram illustrating in more detail actual products of each component included in the position tracking apparatus 100 for head and neck IGS using a magnetic field illustrated in FIG. 1;
FIG. 3 is a diagram illustrating in more detail front and rear portions of a main body part 110;
FIG. 4 is a diagram illustrating in more detail the front, side, and rear portions of a field generator 120;
FIG. 5 is a diagram illustrating an operating system diagram of the main body 110 according to an embodiment of the present disclosure;
FIG. 6 is a schematic diagram illustrating an insulation structure of the main body part 110 according to an embodiment of the present disclosure;
FIG. 7 is a schematic diagram illustrating a software structure of the main body part 110 according to an embodiment of the present disclosure;
FIG. 8 is a schematic diagram illustrating an algorithm structure of the main body part 110 according to an embodiment of the present disclosure;
FIG. 9 is a screen in which a DICOM image of a patient is output through a display device linked to a dedicated application for position tracking for IGS;
FIG. 10 is a screen in which a window for selecting one of a landmark type and a surface type through the display device is output;
FIG. 11 is a screen in which a window for guiding point registration is output through a display device when the landmark type is selected;
FIG. 12 is a screen in which a window for guiding surface registration is output through the display device when the surface type is selected;
FIG. 13 is a screen in which a window for guiding a preset path during the surface registration is output through the display device;
FIG. 14 is a screen output when an EM pointer 140 is positioned within or out of an effective range of the AC magnetic field through the display device in the main body part 110;
FIG. 15 is a screen in which a window for performing surface limit setting of a patient video is output through the display device;
FIG. 16 is a screen in which a window for controlling movement of a patient image in the patient video is output through the display device;
FIG. 17 is a screen in which simultaneous output is performed in the form of an omnidirectional slice plane through volume decomposition of a patient in the patient video through the display device;
FIG. 18 is a screen in which the screen is divided and output according to the screen configuration and number desired by a medical practitioner through the display device;
FIG. 19 is a screen in which a window for adjusting a screen contrast is output according to a contrast ratio desired by the medical practitioner through the display device;
FIG. 20 is a screen in which the position of the pre-registered EM pointer 140 is output according to a desired view mode through the display device;
FIG. 21 is a screen in which results of measuring a distance or size between two points specified through the display device are output; and
FIG. 22 is a screen in which an alarm is generated when the position of the EM pointer 140 gets close to a pre-registered point through the display device.

### DETAILED DESCRIPTION OF THE PREFERRED EMBODIMENT

Hereinafter, embodiments of the present disclosure will be described in detail with reference to the accompanying drawings. However, in the following description, when there is a risk of unnecessarily obscuring the gist of the present disclosure, specific descriptions of widely known functions or configurations will be omitted.

In the attached drawings, identical or corresponding components are given the same reference numerals. In addition, in the description of the embodiments below, redundant description of identical or corresponding components may be omitted. However, even if the description of the components is omitted, it is not intended that such components are not included in any embodiment.

Various advantages and features of disclosed embodiments and methods accomplishing them will become apparent from the following description of embodiments with reference to the accompanying drawings. However, the present disclosure is limited to embodiments disclosed below, but may be implemented in various different forms. These embodiments will be provided only in order to make the invention of the present disclosure complete and allow those skilled in the art to which the present disclosure pertains to completely recognize the scope of the present disclosure.

After terms used in the present specification are briefly described, the present disclosure will be described in detail. General terms that are currently widely used are selected as terms used in embodiments in consideration of functions in the present specification but may be changed depending on the intention of those skilled in the art or a judicial precedent, the emergence of a new technique, and the like. In addition, in a specific case, terms arbitrarily chosen by an applicant may exist. In this case, the meaning of such terms will be mentioned in detail in a corresponding description portion of the present disclosure. Therefore, the terms used in the present disclosure should be defined on the basis of the meaning of the terms and the contents throughout the present disclosure rather than simple names of the terms.

In the present specification, singular forms include plural forms unless the context clearly indicates otherwise. In addition, plural forms include singular forms unless the context clearly indicates otherwise. Throughout the specification, unless otherwise specified, "including" any component means that other components may be further included rather than excluding other components.

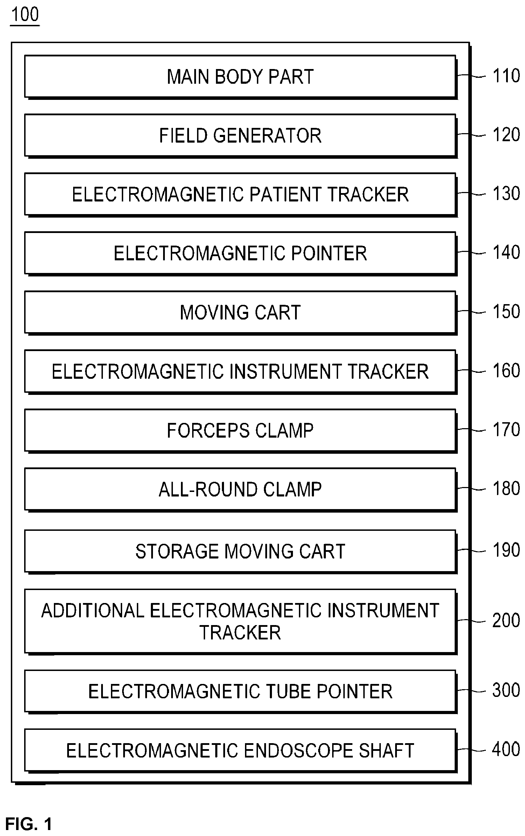

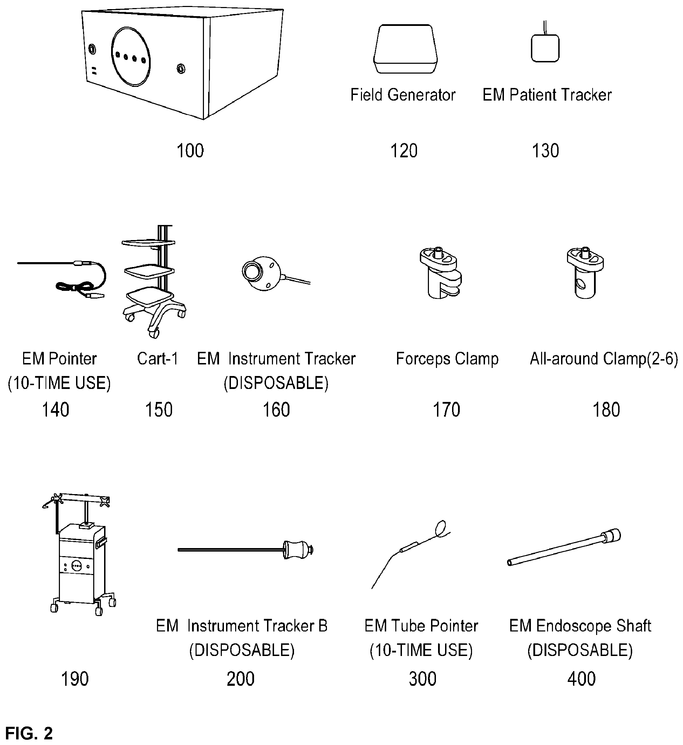

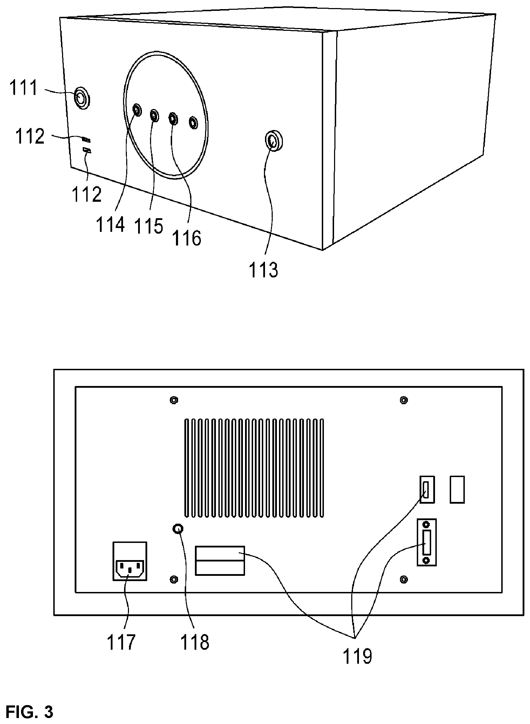

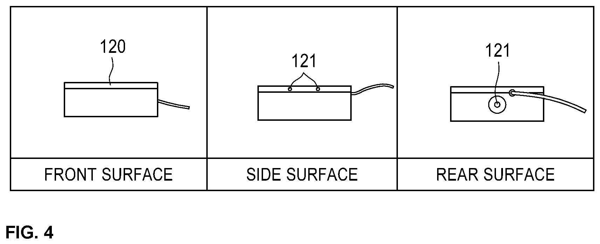

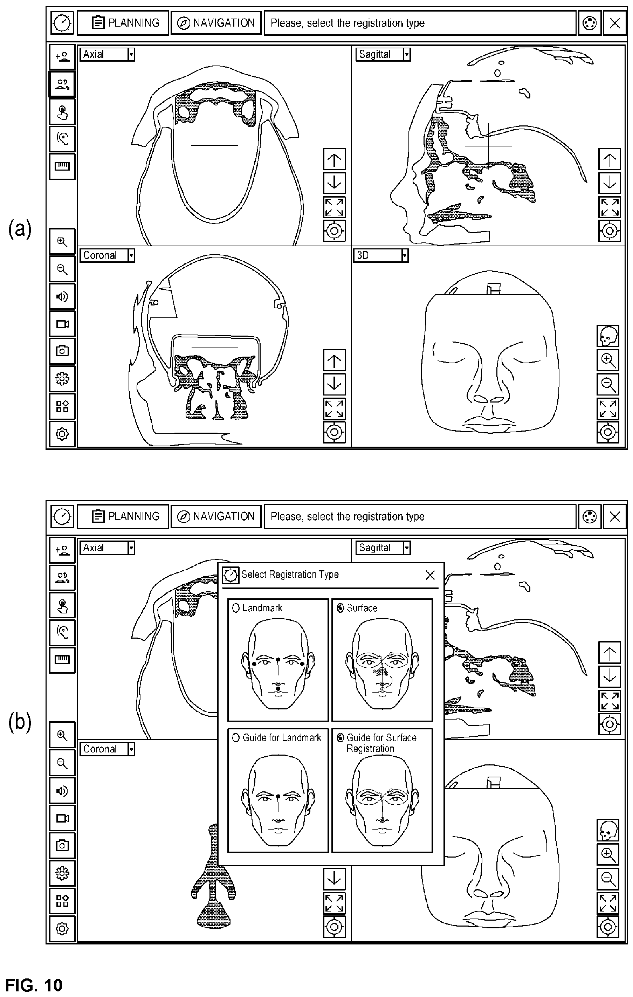

FIG. 1 is a diagram illustrating a configuration of a position tracking apparatus 100 for head and neck IGS using a magnetic field according to an embodiment of the present disclosure, FIG. 2 is a diagram illustrating in more detail actual products of each component included in the position tracking apparatus 100 for head and neck IGS using a magnetic field illustrated in FIG. 1, FIG. 3 is a diagram illustrating in more detail front and rear portions of a main body part 110, and FIG. 4 is a diagram illustrating in more detail the front, side, and rear portions of a field generator 120.

Referring to FIGS. 1 to 4, the position tracking apparatus 100 for head and neck IGS using a magnetic field according to an embodiment of the present disclosure may largely include a main body part 110, a field generator 120, an electromagnetic (EM) patient tracker 130, an EM pointer 140, a moving cart 150, an EM instrument tracker 160, a forceps clamp 170, an all-round clamp 180, and a storage moving cart 190.

In addition, in an embodiment, the position tracking apparatus 100 may further include an additional EM instrument tracker 200, an EM tube pointer 300, and an EM endoscope shaft 400.

The main body part 110 may be electrically connected to the field generator 120, the EM patient tracker 130, the EM pointer 140, the EM instrument tracker 160, the additional EM instrument tracker 200, the EM tube pointer 300, and the EM endoscope shaft 400. As the EM pointer 140 generate an induced current in response to an alternating (AC) magnetic field generated externally by the field generator 120, when the EM patient tracker 130 worn on a patient's head and neck in IGS transmits a detection result signal based on the induced current, the main body part 110 may calculate a distance, position, and direction of the EM pointer 140 within the patient's head and neck in IGS, and map an image generated by calculating the distance, position, and direction of the EM pointer 140 to a previously captured facial X-ray image of the corresponding patient and output the mapped image through a display device connected to the main body part 110.

A front portion of the main body part 110 may be provided with a power switch 111, at least two USB 3.0 connection ports 112 into which a USB memory device is inserted, a connection port 113 for a field generator into which a cable terminal of the field generator 120 is inserted, a connection port 114 for an EM patient tracker into which a cable terminal of the EM patient tracker 130 is inserted, a connection port 115 for an EM pointer into which a cable terminal of the EM pointer 140 is inserted, and a connection port 116 for an EM tube pointer into which a cable terminal of the EM tube pointer is inserted.

In addition, a rear portion of the main body part 110 may be provided with a power grid 117, a grounding potential equalization terminal 118, and an additional connection port 119 including a USB 2.0 connection port, a DVI connection port, and an HDMI connection port.

The main body part 110 will be described in more detail as follows.

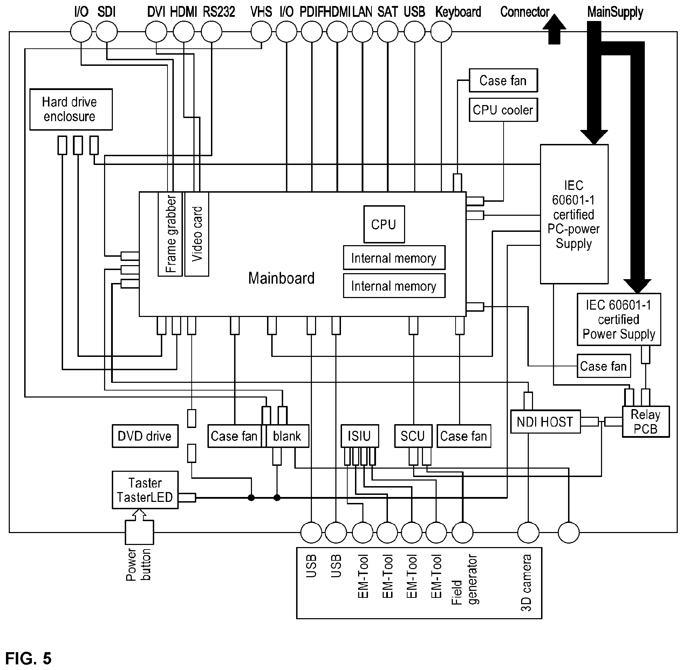

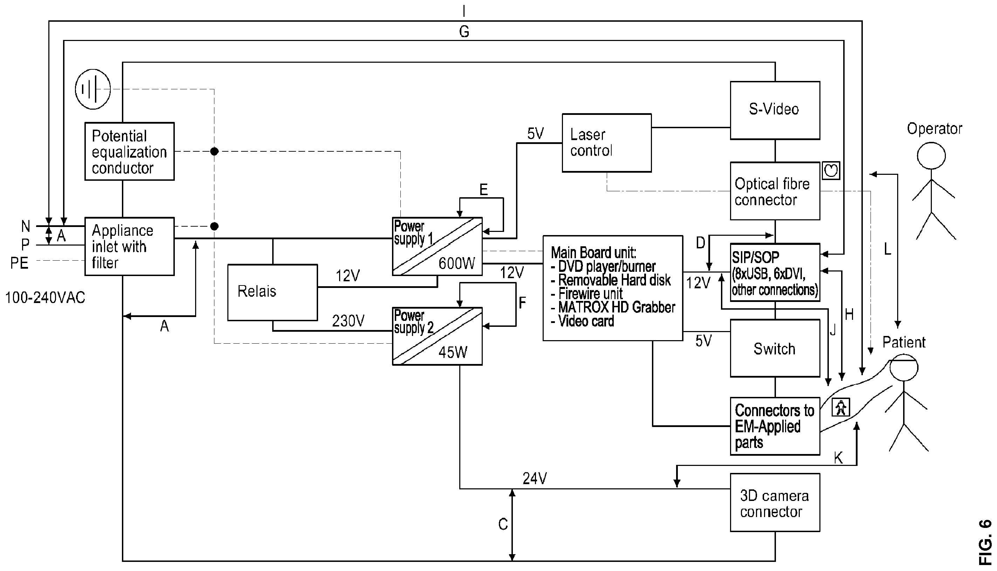

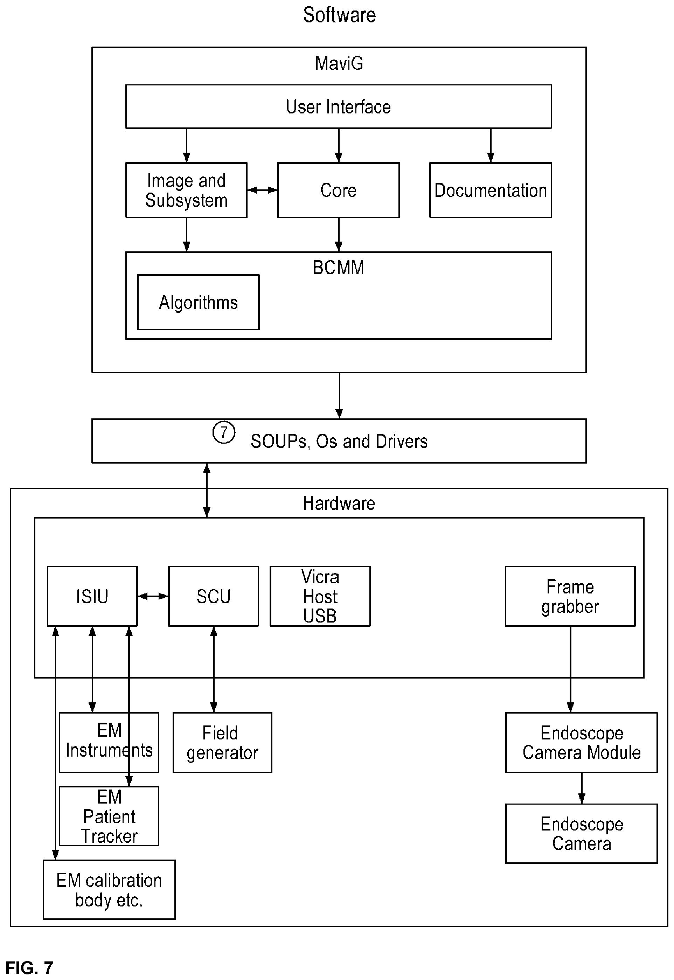

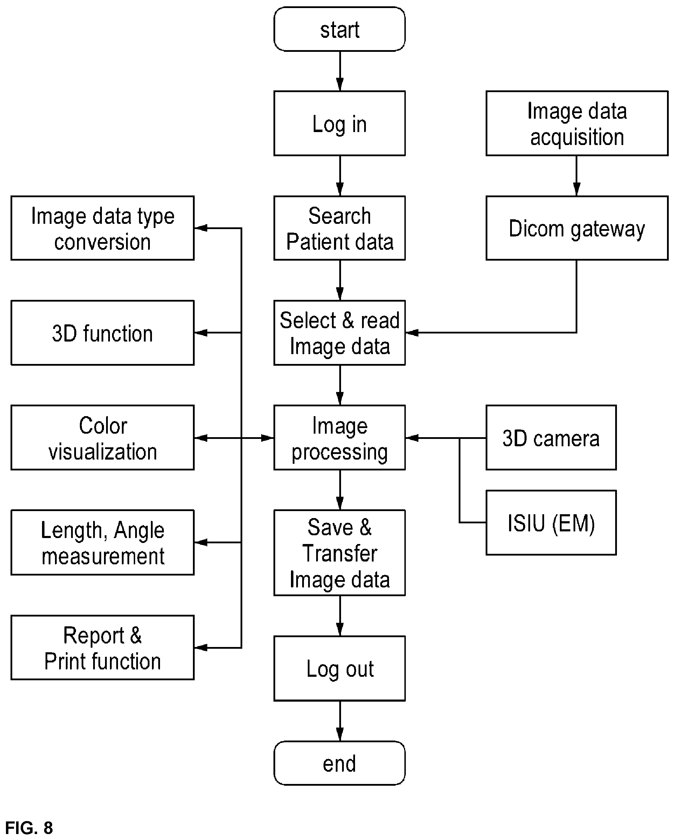

FIG. 5 is a diagram illustrating an operating system diagram of the main body 110 according to an embodiment of the present disclosure, FIG. 6 is a schematic diagram illustrating an insulation structure of the main body part 110 according to an embodiment of the present disclosure, FIG. 7 is a schematic diagram illustrating a software structure of the main body part 110 according to an embodiment of the present disclosure, and FIG. 8 is a schematic diagram illustrating an algorithm structure of the main body part 110 according to an embodiment of the present disclosure.

Referring to FIGS. 5 to 8, the main body part 110 has a rated voltage of 100 to 240 V and 50/60 Hz and an electrical rating of 300 VA power consumption, and includes two fuses of 2.5 A and 205 V, so when an overload occurs, a fuse in a power input terminal may be short-circuited, thereby cutting off the power supply to the main body part 110.

The main body part 110 may include a main board that serves as a logical and physical (accessory function) connection to important electronic components of a system, a CPU that serves to process position information from external devices and perform application calculation processing, a CPU cooler, an internal memory that serves to temporarily save and wait for real-time external position information, a video card that serves to output a radiological video image and image information of a surgical instrument position, a hard drive that serves as a main S/W and surgical area structure and pointer positioning video image storage, a hard drive enclosure that serves as a surgery-related backup data storage protection external function, a DVD drive, a medical PC power supply unit that serves to supply power, a case fan that serves to exchange air to prevent heat generated internally, a serial-to-USB-converter that serves to transmit surgical area video data using USB communication, a power switch that serves to turn on/off an internal power supply, a frame grabber that serves to transmit a signal for the main body S/W and capture an analog signal of a 3D camera in an individual digital unit for S-video transmission, a medical power supply unit that serves to supply 24V power to the 3D camera, an NDI-HOST that serves to transmit and receive data between the 3D camera and the main system, amplify signals, and distribute power to the 3D camera, accessory ports that are ports connected to various pointers, a relay PCB that serves as an interrupt for the power switch, an SCU that serves to calculate a position and direction of the sensor using data collected by an intermediate stage interface unit (ISIU) module, and an ISIU amplification module that serves to amplify an electrical signal generated from a sensor unit.

In addition, in an embodiment, the main body part 110 may amplify the detection result signal transmitted from the EM patient tracker 130 using the ISIU amplification module, may preprocess, in the SCU, the detection result signal amplified by the ISIU amplification module and then may process the preprocessed detection result signal into three-dimensional position information, and may transmit the processed signal to a video output module to be output through the display device.

In addition, in an embodiment, the main body part 110 may include a user interface software module that serves to exchange input from a user and usage information, an image and subsystem software module that serves as communication management module for image devices, a core software module that serves as general processing of patient information and navigation data through DICOM, a documentation software module that serves to a module for storing screen shot and video information, a BCMM software module that serves as a class and utility function for communication with peripheral devices, an algorithms software module that serves to manage each algorithm related to an overall navigation operation, a SOUPs, OSs and drivers software module that serves to transmit data processed through BCMM to the OS for application and performing 3D rendering through an OpenGL driver, an ISIU software module that serves as communication support with external instruments, an SCU software module that serves as communication support with a field generator, a host USB Vicra software module that serves as communication support with a 3D camera, a frame grabber software module that serves as capture management for endoscope camera images, an EM Instruments software module that transmits an electromotive force signal induced in the AS magnetic field to a main body to track a position, an EM patient tracker software module that transmits the induced electromotive force signal in the AS magnetic field to the main body to track a position of a frontal lobe, an EM calibration body **etc.,** software module that serves as an initial registration of EM-related objects (instruments), a field generator that serves to generate an AS magnetic field around the affected area, an endoscope camera module software module that serves to process the image information of the endoscope device and output the processed image information as a digital video signal when a user uses the endoscope, and an endoscope camera software module that serves to manually capture the image of the endoscope device when the user uses the endoscope.

In an embodiment, the field generator 120 is connected to the main body part 110, and may generate an AC magnetic field externally around the affected area through an internal coil according to a control signal transmitted from the main body part 110.

In addition, the EM patient tracker 130 has one side worn on the patient's head and neck in IGS while the other side is connected to the main body part 110, and may detect the AC magnetic field and transmit a detection result signal to the main body part 110.

In addition, in an embodiment, a medical adhesive pad is disposed on a lower side surface of the EM patient tracker 130, and the EM patient tracker 130 is attached to a patient's forehead through adhesive strength of the medical adhesive pad.

In this case, when the EM patient tracker 130 is attached to the patient's forehead through the medical adhesive pad, the main body part 110 may output a screen for selecting one of a landmark type or a surface type through the display device to measure a registration value. This will be described below.

In addition, in an embodiment, the EM pointer 140 has one side inserted into the patient's head and neck in IGS while the other side is connected to the main body part 110, and may generate the induced current in response to the AC magnetic field to notify the position within the affected area. In addition, in an embodiment, the moving cart 150 has the main body part 110 and the field generator 120 mounted thereon and is configured to be movable.

In addition, in an embodiment, the EM instrument tracker 160 is configured such that one side is connected to the main body part 110 and the other side is connected to a commercial surgical instrument, and may guide the commercial surgical instrument inserted into the patient's head and neck during IGS to generate the induced current in response to the AC magnetic field. Therefore, even if there is no dedicated EM pointer 140, the position can be identified in the same way as the dedicated EM pointer 140 by utilizing the EM instrument tracker 160 for the commercial surgical instrument.

In addition, in an embodiment, the forceps clamp 170 is fixed to the moving cart 150, and has one side connected to surgical forceps to fix the forceps, and the all-round clamp 180 is fixed to the moving cart 150, and may clamp and fix the commercial surgical instrument.

Here, the all-round clamp 180 may be provided in various sizes such as a small all-round clamp 181 that clamps the commercial surgical instrument having a diameter of 2 mm to 6 mm and fixes the commercial surgical instrument to the moving cart 150; a medium all-round clamp 182 that clamps the commercial surgical instrument having a diameter of 6 mm to 10 mm and fixes the commercial surgical instrument to the moving cart 150; and a large all-round clamp 183 that clamps the commercial surgical instrument having a diameter of 10 mm to 16 mm and fixes the commercial surgical instrument to the moving cart 150.

In addition, in an embodiment, the storage moving cart 190 may be provided with a storage space for storing the EM patient tracker 130, the EM pointer 140, the EM instrument tracker 160, the forceps clamp 170, and the all-round clamp 180, and may be configured to be movable. For this purpose, a plurality of wheels may be provided on a lower side of the storage moving cart 190.

In addition, in an embodiment, the additional EM instrument tracker 200 is configured such that one side is connected to the main body part 110 and the other side is connected to a commercial balloon catheter, and may guide the commercial balloon catheter inserted into the patient's head and neck during IGS to generate the induced current in response to the AC magnetic field. Therefore, even if there is no dedicated EM pointer 140, the position can be identified in the same way as the dedicated EM pointer 140 by utilizing the EM instrument tracker 160 for the commercial balloon catheter.

In addition, in an embodiment, the EM tube pointer 300 may have one side inserted into the patient's head and neck in IGS while the other side is connected to the main body part 110, generate the induced current in response to the AC magnetic field to notify a position within the head and neck during IGS, and suck foreign substances within the head and neck during IGS through a suction hole formed at a center.

In addition, in an embodiment, the EM endoscope shaft 400 has one side inserted into the patient's head and neck in IGS while being coupled with a commercial endoscope, and may generate the induced current in response to the AC magnetic field to notify the position of the commercial endoscope within the head and neck during IGS.

Next, a process for tracking and identifying the position of the EM pointer 140 positioned within the patient's head and neck during IGS using the position tracking apparatus 100 for head and neck IGS using a magnetic field as described above will be described.

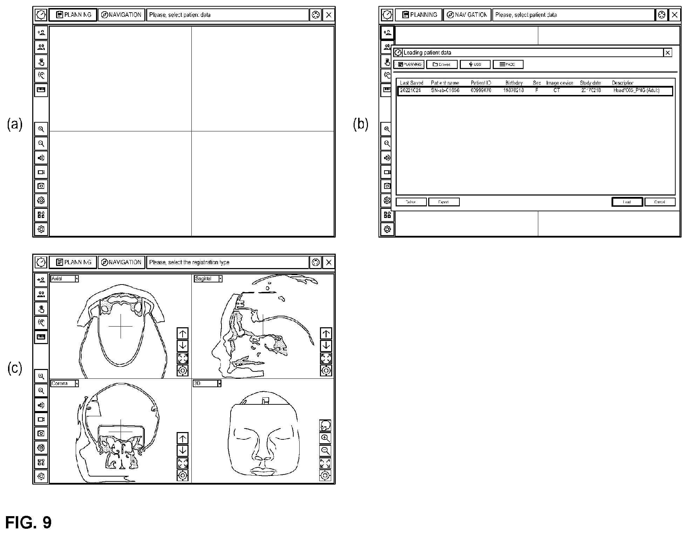

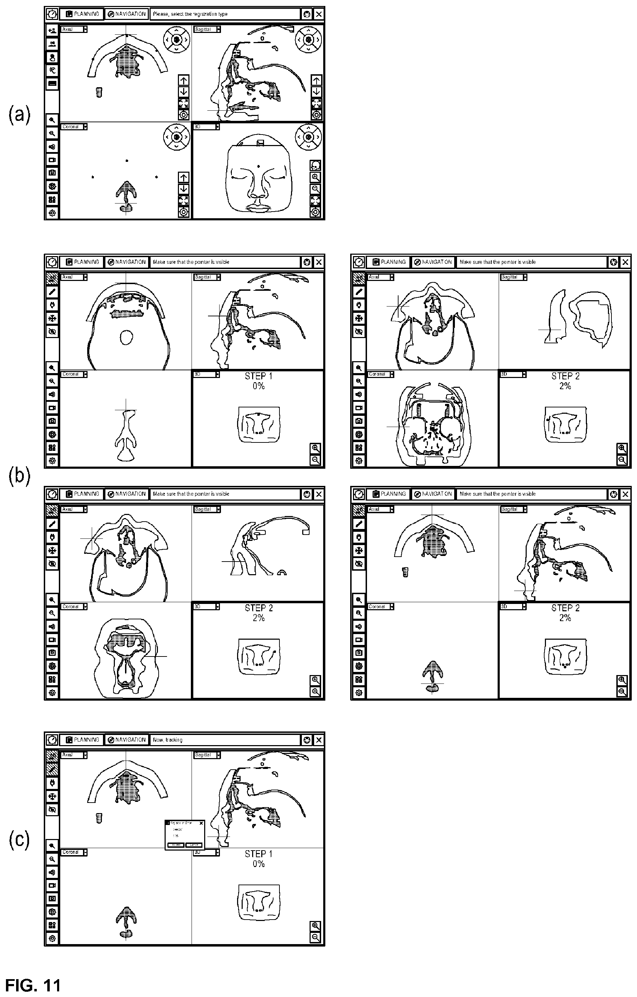

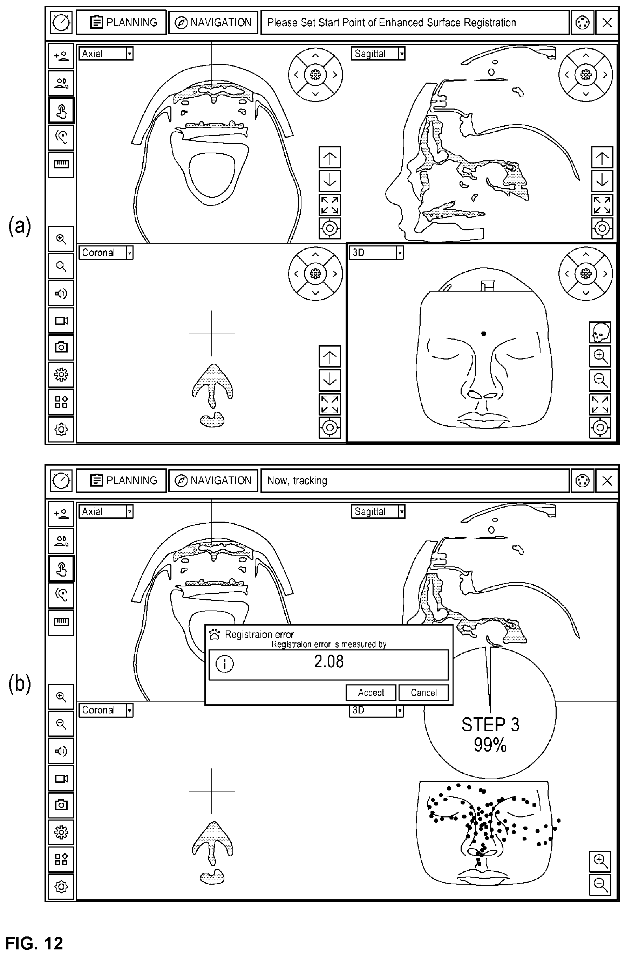

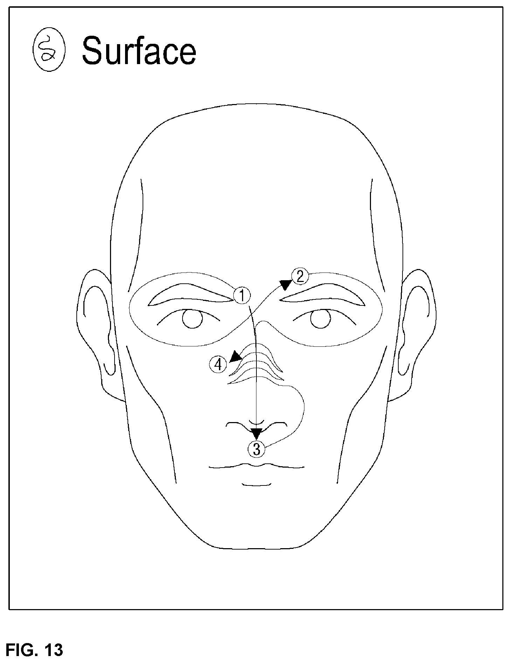

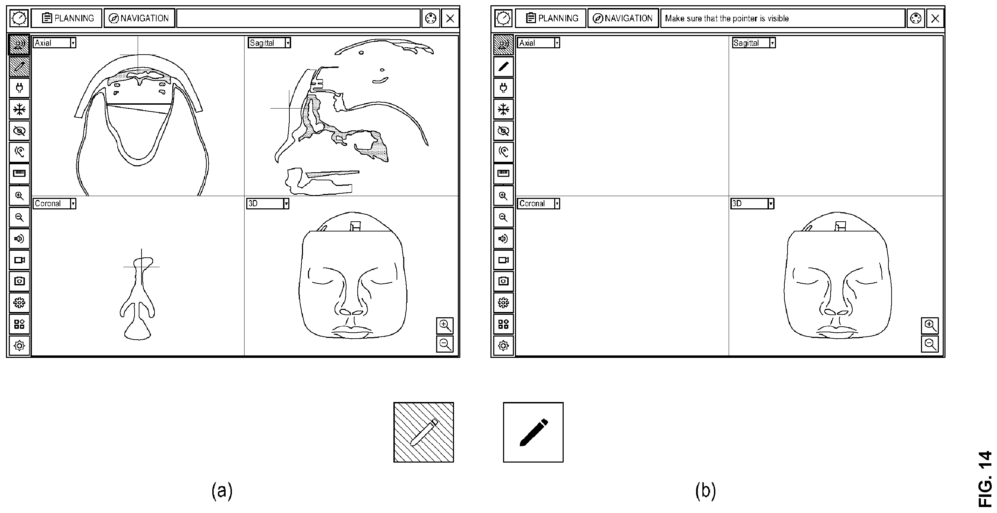

FIG. 9 is a screen in which a DICOM image of a patient is output through a display device linked to a dedicated application for position tracking for IGS, FIG. 10 is a screen in which a window for selecting one of a landmark type and a surface type through the display device is output, FIG. 11 is a screen in which a window for guiding point registration is output through a display device when the landmark type is selected, FIG. 12 is a screen in which a window for guiding surface registration is output through the display device when the surface type is selected, FIG. 13 is a screen in which a window for guiding a preset path during the surface registration is output through the display device, and FIG. 14 is a screen output when the EM pointer 140 is positioned within or out of an effective range of the AC magnetic field through the display device in the main body part 110.

Referring to FIG. 9, when the power switch 111 is operated in an on state by a medical practitioner, the main body part 110 may execute a dedicated application for position tracking for the IGS, retrieve image data for the corresponding patient based on unique ID information for each patient, and then output a DICOM image to the display device.

In this case, the main body part 110 may retrieve the image data for the corresponding patient, and then output a notification to the medical practitioner through the display device to recheck whether the unique ID information and information on a patient to be operated match, thereby preventing the possibility of serious medical accidents that may occur due to the mismatch between the patient and unique ID.

Referring to FIG. 10, according to an embodiment of the present disclosure, when the EM patient tracker 130 is attached to the patient's forehead through the medical adhesive pad, the main body part 110 may output a screen for selecting one of a landmark type or a surface type through the display device to measure a registration value.

In this process, when the landmark type is selected by the medical practitioner, as illustrated in FIG. 11, the main body part 110 may output a guide screen for designating points as a reference point on the display screen, and guide the medical practitioner to move the position of the EM pointer 140 through the guide screen so that the designated point positions are sequentially contacted.

For example, after designating a reference point on the guide screen, the medical practitioner moves the EM pointer 140 and guides the pointer to contact the designated point positions sequentially until the registration value reaches 0% to 100%.

In this process, the main body part 110 may guide a process of guiding the position movement of the EM pointer 140 to be repeatedly performed until the registration value reaches a preset registration value.

In addition, referring to FIG. 12, when the surface type is selected by the medical practitioner, the main body part 110 may output a guide screen for designating starting points on the display screen, and guide the medical practitioner to move the position of the EM pointer 140 through the guide screen so that the EM pointer 140 comes into contact with a patient's face surface along a preset path starting from the starting point.

For example, after designating a starting point as a reference on the guide screen, the medical practitioner is guided to bring the EM pointer 140 into contact with the starting point of the patient's face surface and then move the EM pointer 140 along the preset path while maintaining contact with the patient's face surface until the registration value reaches 100%.

In this process, the main body part 110 may guide a process of guiding the position movement of the EM pointer 140 to be repeatedly performed until the registration value reaches a preset registration value.

In addition, in an embodiment, in the process of guiding the medical practitioner to move the position of the EM pointer 140 through the guide screen so that the EM pointer 140 is contacted along the preset path starting from the starting point on the patient's face surface, as illustrated in FIG. 13, the main body part 110 may designate an area between the patient's forehead and eyebrows as the starting point, and then guide a first path that connects left eye area and right eye area of the patient in a figure-eight shape, a second path that crosses a patient's nose and leads to a philtrum, and a third path that starts from the philtrum and is raised to a glabella while zigzagging across a bridge of the nose through the guide screen. In this case, the first to third paths may be changed at any time.

In addition, in an embodiment, when the EM pointer 140 is positioned within an effective range of the AC magnetic field generated externally through the field generator 120 within the patient's head and neck during IGS, as illustrated in FIG. 14A, the main body part 110 may display a green icon on the screen of the display device, and when the EM pointer 140 is determined to be out of the effective range of the AC magnetic field within the patient's head and neck in IGS, as illustrated in FIG. 14B, the main body part 110 may display a red icon and output a warning notification screen to notify that the EM pointer 140 is out of the effective range on the screen of the display.

In addition, in an embodiment, the position tracking apparatus 100 for head and neck IGS using a magnetic field according to the present disclosure may provide various functions required in the head and neck IGS process in addition to the functions discussed above, which will be described as follows.

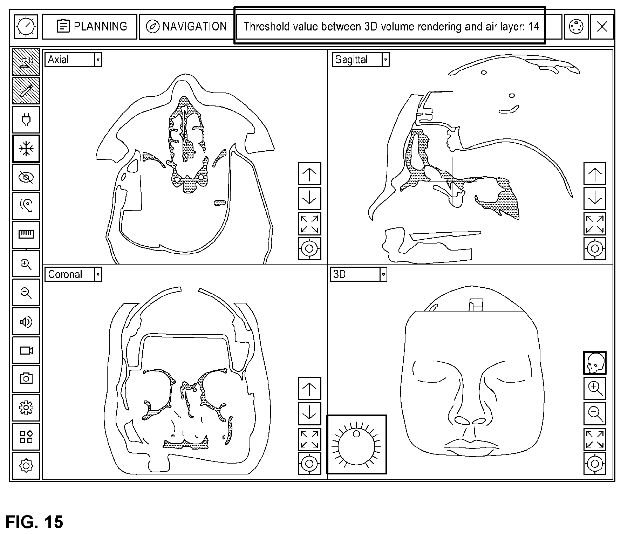

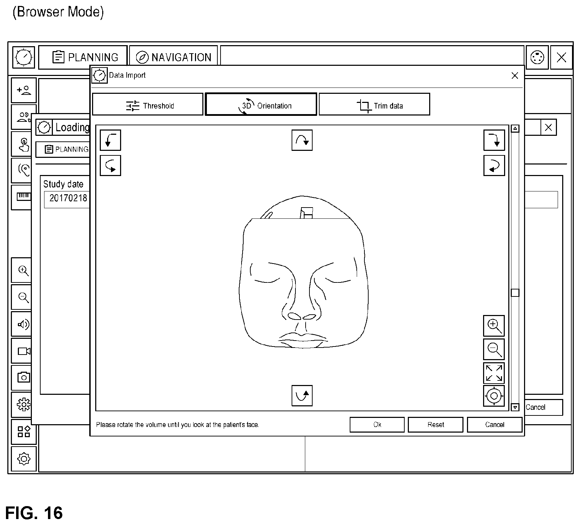

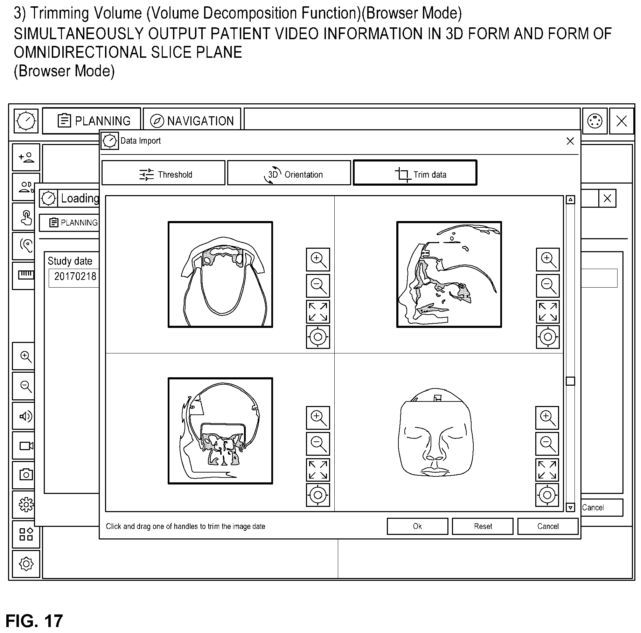

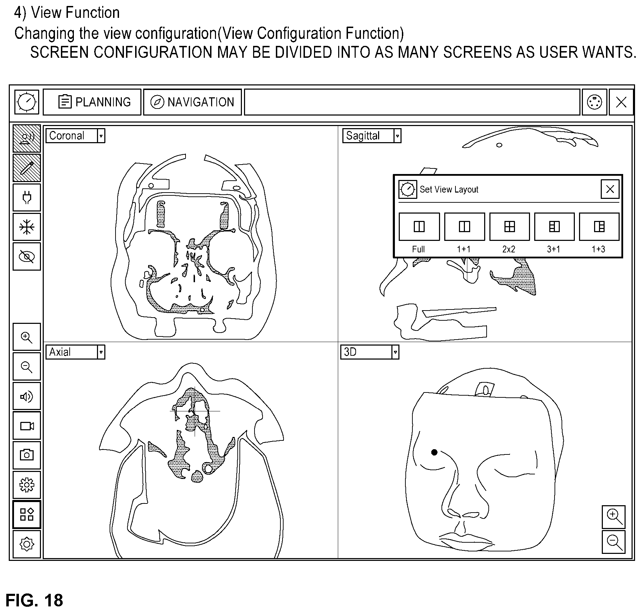

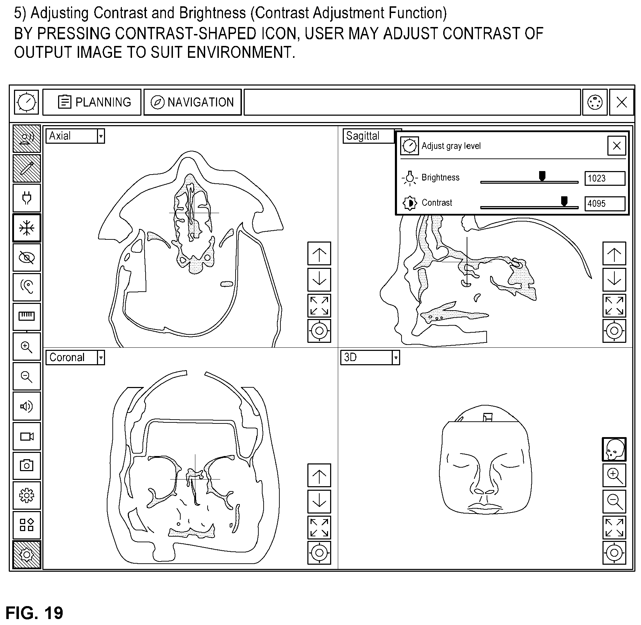

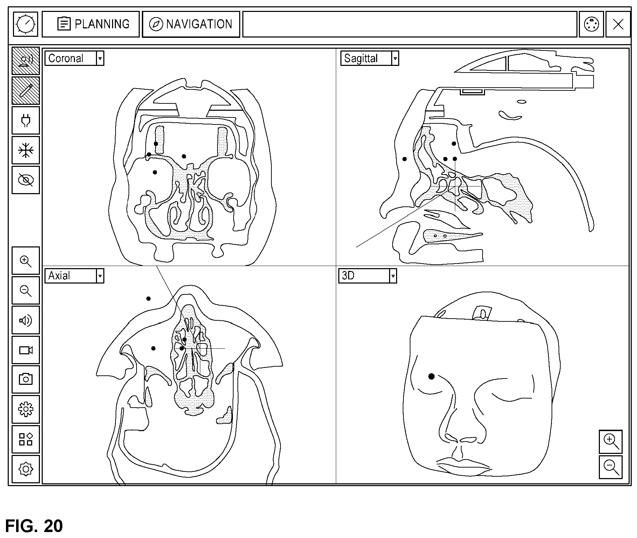

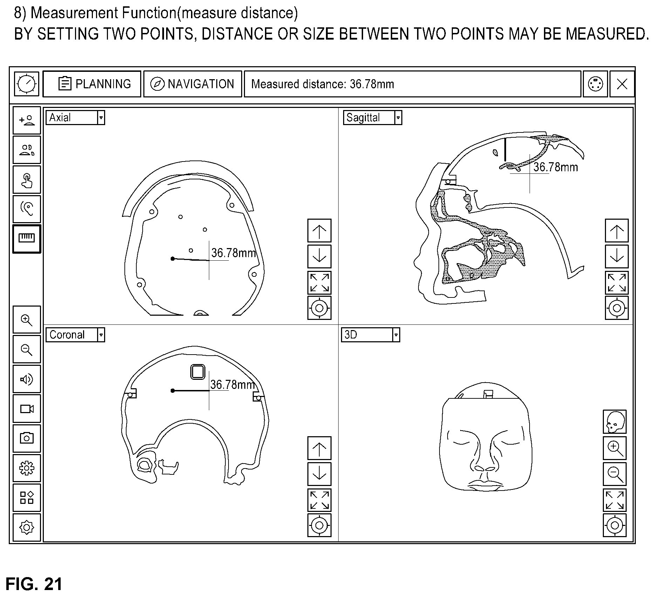

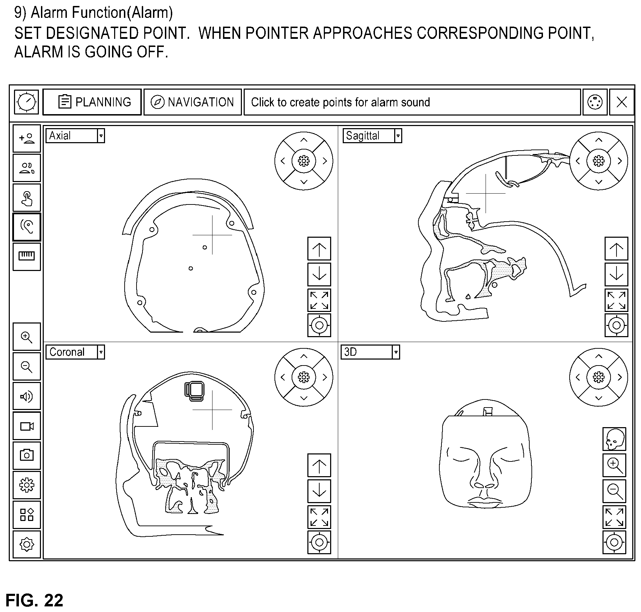

FIG. 15 is a screen in which a window for performing surface limit setting of a patient video is output through the display device, FIG. 16 is a screen in which a window for controlling movement of a patient image in the patient video is output through the display device, FIG. 17 is a screen in which simultaneous output is performed in the form of an omnidirectional slice plane through volume decomposition of a patient in the patient video through the display device, FIG. 18 is a screen in which the screen is divided and output according to the screen configuration and number desired by a medical practitioner through the display device, FIG. 19 is a screen in which a window for adjusting a screen contrast is output according to a contrast ratio desired by the medical practitioner through the display device, FIG. 20 is a screen in which the position of the pre-registered EM pointer 140 is output according to a desired view mode through the display device, FIG. 21 is a screen in which results of measuring a distance or size between two points specified through the display device are output, and FIG. 22 is a screen in which an alarm is generated when the position of the EM pointer 140 gets close to a pre-registered point through the display device.

Referring to FIGS. 15 to 22, the position tracking apparatus 100 for head and neck IGS using a magnetic field according to an embodiment of the present disclosure may clearly delineate the space between the patient's skin surface and the air layer represented in the video as illustrated in FIG. 15, thereby minimizing and outputting artifacts that occur in the video. In this process, when the medical practitioner presses a Freeze navigation button on the upper left of the screen to fix the screen and then selects a surface limit setting button, as a dial for adjusting the skin surface is provided, the medical practitioner may change the surface limit setting as desired by manipulating the dial.

In addition, the position tracking apparatus 100 for head and neck IGS using a magnetic field according to an embodiment of the present disclosure may provide adjust orientation (direction adjustment function) as illustrated in FIG. 16, so the medical practitioner may adjust the movement of the 3D patient video, click the direction in which he or she wants to move, and then adjust a slider, thereby providing an observation of an appearance other than the front.

In addition, the position tracking apparatus 100 for head and neck IGS using a magnetic field according to an embodiment of the present disclosure may provide a trimming volume (volume decomposition function) as illustrated in FIG. 17 to simultaneously output and provide patient video information in a 3D form and a shape of the slice plane in all directions.

In addition, the position tracking apparatus 100 for head and neck IGS using a magnetic field according to an embodiment of the present disclosure may provide changing the view configuration (view configuration function) as illustrated in FIG. 18, thereby allowing the medical practitioner to divide the screen configuration into the desired number of screens and output the screens

In addition, the position tracking apparatus 100 for head and neck IGS using a magnetic field according to an embodiment of the present disclosure may provide adjusting contrast and brightness (contrast adjustment function) as illustrated in FIG. 19, thereby allowing a medical practitioner to adjust the contrast to suit a desired environment for a patient video in a 3D form.

In addition, the position tracking apparatus 100 for head and neck IGS using a magnetic field according to an embodiment of the present disclosure may merge the pointer instrument and the patient video as illustrated in FIG. 20 to check the position of the EM pointer 140 in the video screen in real time when the EM pointer 140 is inserted into the human body.

In addition, the position tracking apparatus 100 for head and neck IGS using a magnetic field according to an embodiment of the present disclosure may provide a measurement function (measure distance) as illustrated in FIG. 21, thereby measuring the distance or size between two specific points and then providing the measurement result.

In addition, the position tracking apparatus 100 for head and neck IGS using a magnetic field according to an embodiment of the present disclosure may provide the alarm function as illustrated in FIG. 22 to generate an alarm when the position of the EM pointer 140 approaches a pre-registered point, thereby allowing the medical practitioner to recognize the alarm.

While the embodiments described above have been described as utilizing the aspects of the present disclosure in one or more standalone computer systems, the present disclosure is not limited thereto, and may be implemented in conjunction with any computing environment, such as a network or a distributed computing environment. Furthermore, the aspects of the present disclosure may be implemented in a plurality of processing chips or devices, and storage may be similarly affected across a plurality of devices. Such devices may include PCs, network servers, and portable devices.

While the present disclosure has been described in connection with some embodiments herein, various modifications and changes can be made without departing from the scope of the present disclosure, which can be understood by a person skilled in the art to which the invention belongs. Furthermore, such modifications and variations are intended to fall within the scope of the claims appended to the present specification.

### Reference number list

FIG. 1
100: POSITION TRACKING APPARATUS FOR HEAD AND NECK IMAGE GUIDED SURGERY (IGS)
100 -> 110: MAIN BODY PART
111: POWER SWITCH
112: USB 3.0 CONNECTION PORTS
113: CONNECTION PORT FOR THE FIELD GENERATOR 120
114: CONNECTION PORT FOR THE EM PATIENT TRACKER 130
115: CONNECTION PORT FOR THE EM POINTER 140
116: CONNECTION PORT FOR THE EM TUBE POINTER 300
117: POWER GRID
118: GROUNDING POTENTIAL EQUALIZATION TERMINAL
119: CONNECTION PORTS, WHICH MAY INCLUDE A USB 2.0 CONNECTION PORT, A DVI CONNECTION PORT, AND AN HDMI CONNECTION PORT
110 -> 120: FIELD GENERATOR
121: FIXED SUPPORT HOLE
120 -> 130: ELECTROMAGNETIC PATIENT TRACKER
130 -> 140: ELECTROMAGNETIC POINTER
140 -> 150: MOVING CART
150 -> 160: ELECTROMAGNETIC INSTRUMENT TRACKER
160 -> 170: FORCEPS CLAMP
170 -> 180: ALL-ROUND CLAMP
180: SOUND OUTPUT MODULE
190: STORAGE MOVING CART
200: ADDITIONAL ELECTROMAGNETIC INSTRUMENT TRACKER
300: ELECTROMAGNETIC TUBE POINTER
400: ELECTROMAGNETIC ENDOSCOPE SHAFT

## Claims

1. A position tracking apparatus for head and neck image guided surgery (IGS) (100) using a magnetic field, comprising:
a main body part (110);
a field generator (120) that is connected to the main body part (110) and generates externally an alternating (AC) magnetic field around an affected area through an internal coil according to a control signal transmitted from the main body part (110);
an electromagnetic (EM) patient tracker (130) that has one side worn on a patient's head and neck in IGS while the other side is connected to the main body part (110) and detects the AC magnetic field and transmits a detection result signal to the main body part (110);
an EM pointer (140) that has one side inserted into the patient's head and neck in IGS while the other side is connected to the main body part (110) and generates an induced current in response to the AC magnetic field to notify a position within the affected area;
a moving cart (150) that has the main body part (110) and the field generator (120) mounted thereon and is configured to be movable;
an EM instrument tracker (160) that is configured such that one side is connected to the main body part (110) and the other side is connected to a commercial surgical instrument, and guides the commercial surgical instrument inserted into the patient's head and neck in IGS to generate the induced current in response to the AC magnetic field;
a forceps clamp (170) that is fixed to the moving cart (150) and has one side designed to fix surgical forceps;
an all-round clamp (180) that is fixed to the moving cart (150) and clamps and fixes the commercial surgical instrument; and
a storage moving cart (190) that is provided with a storage space for receiving the EM patient tracker (130), the EM pointer (140), the EM instrument tracker (160), the forceps clamp (170), and the all-round clamp (180), and is configured to be movable,
wherein as the EM pointer (140) generates the induced current in response to the AC magnetic field generated externally by the field generator (120), when the EM patient tracker (130) worn on the patient's head and neck in IGS transmits the detection result signal detecting the induced current, the main body part (110) calculates a distance, position, and direction of the EM pointer (140) within the patient's head and neck in IGS and maps an image generated by calculating the distance, position, and direction of the EM pointer (140) to a previously captured facial X-ray image of the patient and outputs the mapped image through a display device connected to the main body part (110), **characterized by** a fixed support hole (121) is formed on each side and rear surface of the field generator (120) with which a fixed support for mounting on the moving cart (150) or a surgical bed is fastenable or fastened, and the field generator (120) receives power for generating the AC magnetic field from the main body part (110).

2. The position tracking apparatus according to claim 1, wherein a front portion of the main body part (110) is provided with:
a power switch (111);
at least two USB 3.0 connection ports (112) into which a USB memory device is insertable or inserted;
a connection port (113) for a field generator into which a cable terminal of the field generator (120) is insertable or inserted;
a connection port (114) for an EM patient tracker into which a cable terminal of the EM patient tracker (130) is insertable or inserted;
a connection port (115) for an EM pointer into which a cable terminal of the EM pointer (140) is insertable or inserted; and
a connection port (116) for an EM tube pointer into which a cable terminal of the EM tube pointer is insertable or inserted, and
a rear portion of the main body part (110) is provided with:
a power grid (117);
a grounding potential equalization terminal (118); and
an additional connection port (119) that includes a USB 2.0 connection port, a DVI connection port, and an HDMI connection port.

3. The position tracking apparatus according to any one of claims 1 or 2, wherein the main body part (110) amplifies the detection result signal transmitted from the EM patient tracker (130) using an Intermediate stage interface unit (ISIU) amplification module, preprocesses, in an SCU, the detection result signal amplified by the ISIU amplification module and then processes the preprocessed detection result signal into three-dimensional position information, and transmits the processed signal to a video output module to be output through the display device.

4. The position tracking apparatus according to any one of claims 2 to 3, wherein when the power switch (111) is operated in an on state by a medical practitioner, the main body part (110) executes a dedicated application for position tracking for the IGS, retrieves image data for the corresponding patient based on unique ID information for each patient, and then outputs a DICOM image to the display device.

5. The position tracking apparatus according to any one of claims 1 to 4, wherein the main body part (110) retrieves the image data for the corresponding patient, and then outputs a notification to the medical practitioner through the display device to recheck whether the unique ID information and information on a patient to be operated match.

6. The position tracking apparatus according to any one of claims 1 to 5, wherein a medical adhesive pad is disposed on a lower side surface of the EM patient tracker (130), and the EM patient tracker (130) is attached to a patient's forehead through adhesive strength of the medical adhesive pad.

7. The position tracking apparatus according to claim 6, wherein when the EM patient tracker (130) is attached to the patient's forehead through the medical adhesive pad, the main body part (110) outputs a screen for selecting one of a landmark type or a surface type through the display device to measure a registration value.

8. The position tracking apparatus according to claim 7, wherein when the landmark type is selected by the medical practitioner, the main body part (110) outputs a guide screen for designating reference points on the display screen, and guides the medical practitioner to move the position of the EM pointer (140) through the guide screen so that the designated point positions are sequentially contacted.

9. The position tracking apparatus according to any one of claims 7 to 8, wherein when the surface type is selected by the medical practitioner, the main body part (110) outputs a guide screen for designating starting points on the display screen, and guides the medical practitioner to move the position of the EM pointer (140) through the guide screen so that the EM pointer (140) comes into contact with a patient's face surface along a preset path starting from the starting point.

10. The position tracking apparatus according to any one of claims 1 to 9, wherein the main body part (110) guides a process of guiding the position movement of the EM pointer (140) to be repeatedly preformed until the registration value reaches a preset registration value.

11. The position tracking apparatus according to claim 10, wherein in the process of guiding the medical practitioner to move the position of the EM pointer (140) through the guide screen so that the EM pointer (140) is contacted along the preset path starting from the starting point on the patient's face surface, the main body part (110) designates an area between the patient's forehead and eyebrows as the starting point, and then guides a first path that connects left eye area and right eye area of the patient in a figure-eight shape, a second path that crosses a patient's nose and leads to a philtrum, and a third path that starts from the philtrum and is raised to a glabella while zigzagging across a bridge of the nose through the guide screen.

12. The position tracking apparatus according to any one of claims 10 to 11,
wherein when the EM pointer (140) is positioned within an effective range of the AC magnetic field generated externally through the field generator (120) within the patient's head and neck in IGS, the main body part (110) displays a green icon on the screen of the display device, and
when the EM pointer (140) is determined to be out of the effective range of the AC magnetic field within the patient's head and neck in IGS, the main body part (110) displays a red icon and outputs a warning notification screen to notify that the EM pointer (140) is out of the effective range on the screen of the display.

13. The position tracking apparatus according to any one of claims 1 to 12, wherein the all-round clamp (180) includes:
a small all-round clamp (181) that clamps the commercial surgical instrument having a diameter of 2 mm to 6 mm and fixes the commercial surgical instrument to the moving cart (150);
a medium all-round clamp (182) that clamps the commercial surgical instrument having a diameter of 6 mm to 10 mm and fixes the commercial surgical instrument to the moving cart (150); and
a large all-round clamp (183) that clamps the commercial surgical instrument having a diameter of 10 mm to 16 mm and fixes the commercial surgical instrument to the moving cart (150).

14. The position tracking apparatus according to any one of claims 1 to 13, further comprising:
an additional EM instrument tracker (200) that is configured such that one side is connected to the main body part (110) and the other side is connected to a commercial balloon catheter, and guides the commercial balloon catheter inserted into the patient's head and neck in IGS to generate the induced current in response to the AC magnetic field;
an EM tube pointer (300) that has one side inserted into the patient's head and neck in IGS while the other side is connected to the main body part (110), generates the induced current in response to the AC magnetic field to notify a position within the head and neck during IGS, and sucks foreign substances within the head and neck during IGS through a suction hole formed at a center; and
an EM endoscope shaft (400) that has one side inserted into the patient's head and neck in IGS while the other side is coupled to a commercial endoscope, and generates the induced current in response to the AC magnetic field to notify a position of the commercial endoscope within the head and neck during IGS.

## Patentansprüche

1. Eine Vorrichtung (100) zur Positionsverfolgung für die bildgestützte Chirurgie (IGS) an Kopf und Hals unter Verwendung eines Magnetfelds, umfassend:
einen Hauptkörperteil (110);
einen Feldgenerator (120), der mit dem Hauptkörperteil (110) verbunden ist und über eine interne Spule entsprechend einem vom Hauptkörperteil (110) übertragenen Steuersignal extern ein magnetisches Wechselfeld (AC) um einen betroffenen Bereich herum erzeugt;
einen elektromagnetischen (EM) Patienten-Tracker (130), der bei der IGS mit einer Seite am Kopf und Hals eines Patienten getragen wird, während die andere Seite mit dem Hauptkörperteil (110) verbunden ist, und der das Wechselfeld erfasst und ein Erfassungsergebnissignal an den Hauptkörperteil (110) überträgt;
einen EM-Zeiger (140), der mit einer Seite in den Kopf und Hals des Patienten in IGS eingeführt wird, während die andere Seite mit dem Hauptkörperteil (110) verbunden ist, und der als Reaktion auf das Wechselstrom-Magnetfeld einen induzierten Strom erzeugt, um eine Position innerhalb des betroffenen Bereichs anzuzeigen;
einen beweglichen Wagen (150), auf dem der Hauptkörperteil (110) und der Feldgenerator (120) montiert sind und der so konfiguriert ist, dass er beweglich ist;
einen EM-Instrumenten-Tracker (160), der so konfiguriert ist, dass eine Seite mit dem Hauptkörperteil (110) und die andere Seite mit einem handelsüblichen chirurgischen Instrument verbunden ist, und der das in den Kopf und Hals des Patienten unter IGS eingeführte handelsübliche chirurgische Instrument führt, um den induzierten Strom als Reaktion auf das Wechselstrom-Magnetfeld zu erzeugen;
eine Zangenhalterung (170), die an dem beweglichen Wagen (150) befestigt ist und an einer Seite zur Befestigung von chirurgischen Zangen ausgelegt ist;
eine Rundumklemme (180), die an dem beweglichen Wagen (150) befestigt ist und das handelsübliche chirurgische Instrument festklemmt und fixiert; und
einen beweglichen Aufbewahrungswagen (190), der mit einem Stauraum zur Aufnahme des EM-Patienten-Trackers (130), des EM-Zeigers (140), des EM-Instrumenten-Trackers (160), der Zangenklemme (170) und der Rundumklemme (180) versehen ist und so konfiguriert ist, dass er beweglich ist,
wobei, wenn der EM-Zeiger (140) den induzierten Strom als Reaktion auf das vom Feldgenerator (120) extern erzeugte Wechselstrom-Magnetfeld erzeugt, der am Kopf und Hals des Patienten in IGS getragene EM-Patiententracker (130) das den induzierten Strom erfassende Erfassungsergebnissignal überträgt, der Hauptkörperteil (110) eine Entfernung, Position und Richtung des EM-Zeigers (140) innerhalb des Kopfes und Halses des Patienten im IGS berechnet und ein durch die Berechnung der Entfernung, Position und Richtung des EM-Zeigers (140) erzeugtes Bild auf ein zuvor aufgenommenes Röntgenbild des Gesichts des Patienten abbildet und das abgebildete Bild über eine mit dem Hauptkörperteil (110) verbundene Anzeigevorrichtung ausgibt, **gekennzeichnet durch** eine Befestigungsbohrung (121), die an jeder Seiten- und Rückseite des Feldgenerators (120) ausgebildet ist, mit der eine feste Halterung zur Montage an einem beweglichen Wagen (150) oder einem Operationsbett befestigt werden kann oder befestigt ist, und der Feldgenerator (120) wird vom Hauptkörperteil (110) mit Strom zur Erzeugung des Wechselstrommagnetfelds versorgt.

2. Die Vorrichtung zur Positionsverfolgung nach Anspruch 1, wobei ein vorderer Abschnitt des Hauptkörperteils (110) versehen ist mit:
einem Netzschalter (111);
mindestens zwei USB-3.0-Anschlüsse (112), in die ein USB-Speichergerät einsteckbar ist oder eingesteckt ist;
einen Anschluss (113) für einen Feldgenerator, in den ein Kabelanschluss des Feldgenerators (120) einsteckbar ist oder eingesteckt ist;
einen Anschluss (114) für einen EM-Patienten-Tracker, in den ein Kabelanschluss des EM-Patienten-Trackers (130) einsteckbar ist oder eingesteckt ist;
einen Anschluss (115) für einen EM-Zeiger, in den ein Kabelanschluss des EM-Zeigers (140) einsteckbar ist oder eingesteckt ist; und
einen Anschluss (116) für einen EM-Röhrenzeiger, in den ein Kabelanschluss des EM-Röhrenzeigers einsteckbar ist oder eingesteckt ist, und
ein hinterer Abschnitt des Hauptkörperteils (110) ist versehen mit:
einem Stromnetz (117);
einem Erdungspotential-Ausgleichsanschluss (118); und
einem zusätzlichen Anschluss (119), der einen USB-2.0-Anschluss,
einen DVI-Anschluss und einen HDMI-Anschluss umfasst.

3. Die Vorrichtung zur Positionsverfolgung nach einem der Ansprüche 1 oder 2, wobei der Hauptkörperteil (110) das vom EM-Patienten-Tracker (130) übermittelte Erfassungsergebnissignal unter Verwendung eines Verstärkungsmoduls der Zwischenstufen-Schnittstelleneinheit (ISIU) verstärkt, in einer SCU vorverarbeitet, das durch das ISIU-Verstärkungsmodul verstärkte Erfassungsergebnissignal vorverarbeitet und anschließend das vorverarbeitete Erfassungsergebnissignal zu dreidimensionalen Positionsinformationen verarbeitet und das verarbeitete Signal an ein Videoausgabemodul überträgt, um es über die Anzeigevorrichtung auszugeben.

4. Die Vorrichtung zur Positionsverfolgung nach einem der Ansprüche 2 bis 3, wobei, wenn der Netzschalter (111) von einem Arzt in den eingeschalteten Zustand gebracht wird, der Hauptkörperteil (110) eine spezielle Anwendung zur Positionsverfolgung für das IGS ausführt, Bilddaten für den entsprechenden Patienten auf der Grundlage eindeutiger ID-Informationen für jeden Patienten abruft und anschließend ein DICOM-Bild an die Anzeigevorrichtung ausgibt..

5. Die Vorrichtung zur Positionsverfolgung nach einem der Ansprüche 1 bis 4, wobei das Hauptkörperteil (110) die Bilddaten für den entsprechenden Patienten abruft und anschließend über die Anzeigevorrichtung eine Benachrichtigung an den Arzt ausgibt, um erneut zu überprüfen, ob die eindeutigen ID-Informationen und die Informationen zu einem zu operierenden Patienten übereinstimmen.

6. Die Vorrichtung zur Positionsverfolgung nach einem der Ansprüche 1 bis 5, wobei an einer unteren Seitenfläche des EM-Patienten-Trackers (130) ein medizinisches Klebepolster angeordnet ist und der EM-Patienten-Tracker (130) durch die Klebekraft des medizinischen Klebepolsters an der Stirn eines Patienten befestigt wird.

7. Die Vorrichtung zur Positionsverfolgung nach Anspruch 6, wobei der Hauptkörperteil (110) über die Anzeigevorrichtung einen Bildschirm zur Auswahl zwischen einem Orientierungspunkt-Typ und einem Oberflächentyp ausgibt, um einen Registrierungswert zu messen, wenn der EM-Patienten-Tracker (130) mittels des medizinischen Klebepads an der Stirn des Patienten befestigt ist.

8. Die Vorrichtung zur Positionsverfolgung nach Anspruch 7, wobei der Hauptkörperteil (110) einen Führungsbildschirm zur Festlegung von Referenzpunkten auf dem Bildschirm ausgibt und den Arzt anleitet, die Position des EM-Zeigers (140) über den Führungsbildschirm so zu bewegen, dass die festgelegten Punktpositionen nacheinander berührt werden, wenn der Orientierungspunkt-Typ vom Arzt ausgewählt wird.

9. Die Vorrichtung zur Positionsverfolgung nach einem der Ansprüche 7 bis 8, wobei der Hauptkörperteil (110) einen Führungsbildschirm zur Festlegung von Startpunkten auf dem Bildschirm ausgibt und den Arzt anleitet, die Position des EM-Zeigers (140) über den Führungsbildschirm so zu bewegen, dass der EM-Zeiger (140) entlang eines voreingestellten Pfades, der am Startpunkt beginnt, mit der Gesichtsoberfläche eines Patienten in Kontakt kommt, wenn der Arzt den Oberflächentyp auswählt.

10. Die Vorrichtung zur Positionsverfolgung nach einem der Ansprüche 1 bis 9, wobei der Hauptkörperabschnitt (110) einen Vorgang zur Steuerung der Positionsbewegung des EM-Zeigers (140) so steuert, dass dieser wiederholt ausgeführt wird, bis der Registrierungswert einen voreingestellten Registrierungswert erreicht.

11. Die Vorrichtung zur Positionsverfolgung nach Anspruch 10, wobei bei dem Vorgang, bei dem der Arzt angeleitet wird, die Position des EM-Zeigers (140) über den Führungsbildschirm so zu bewegen, dass der EM-Zeiger (140) entlang des voreingestellten Pfades, beginnend am Startpunkt auf der Gesichtshaut des Patienten, berührt wird, der Hauptkörperteil (110) einen Bereich zwischen der Stirn und den Augenbrauen des Patienten als Startpunkt festlegt, und anschließend einen ersten Pfad anzeigt, der den linken Augenbereich und den rechten Augenbereich des Patienten in Form einer Acht verbindet, einen zweiten Pfad, der die Nase des Patienten kreuzt und zum Philtrum führt, sowie einen dritten Pfad, der vom Philtrum ausgeht und zur Glabella hin ansteigt, während er über den Nasenrücken im Zickzack durch den Führungsbildschirm verläuft.

12. Die Vorrichtung zur Positionsverfolgung nach einem der Ansprüche 10 bis 11, wobei der Hauptkörperteil (110) ein grünes Symbol auf dem Bildschirm der Anzeigevorrichtung anzeigt, wenn sich der EM-Zeiger (140) innerhalb eines Wirkbereichs des Wechselstrommagnetfelds befindet, das extern durch den Feldgenerator (120) im Kopf- und Halsbereich des Patienten im IGS erzeugt wird, und
der Hauptkörperteil (110) ein rotes Symbol anzeigt und einen Warnbildschirm ausgibt, um auf dem Bildschirm der Anzeige darauf hinzuweisen, dass sich der EM-Zeiger (140) außerhalb des Wirkbereichs befindet, wenn festgestellt wird, dass sich der EM-Zeiger (140) außerhalb des Wirkbereichs des Wechselstrom-Magnetfelds im Kopf- und Halsbereich des Patienten im IGS befindet.

13. Die Vorrichtung zur Positionsverfolgung nach einem der Ansprüche 1 bis 12, wobei die Rundumklemme (180) umfasst:
eine kleine Rundumklemme (181), die das handelsübliche chirurgische Instrument mit einem Durchmesser von 2 mm bis 6 mm festklemmt und das handelsübliche chirurgische Instrument am beweglichen Wagen (150) befestigt;
eine mittlere Rundumklemme (182), die das handelsübliche chirurgische Instrument mit einem Durchmesser von 6 mm bis 10 mm festklemmt und das handelsübliche chirurgische Instrument am beweglichen Wagen (150) befestigt; und
eine große Allzweckklemme (183), die das handelsübliche chirurgische Instrument mit einem Durchmesser von 10 mm bis 16 mm festklemmt und das handelsübliche chirurgische Instrument am beweglichen Wagen (150) befestigt.

14. Die Vorrichtung zur Positionsverfolgung nach einem der Ansprüche 1 bis 13, die ferner umfasst:
einen zusätzlichen EM-Instrumenten-Tracker (200), der so konfiguriert ist, dass eine Seite mit dem Hauptkörperteil (110) und die andere Seite mit einem handelsüblichen Ballonkatheter verbunden ist, und der den in den Kopf und Hals des Patienten eingeführten handelsüblichen Ballonkatheter im IGS-Modus führt, um als Reaktion auf das Wechselstrom-Magnetfeld den induzierten Strom zu erzeugen;
einen EM-Rohrzeiger (300), der mit einer Seite in den Kopf und Hals des Patienten während der IGS eingeführt ist, während die andere Seite mit dem Hauptkörperteil (110) verbunden ist, der induzierte Strom als Reaktion auf das Wechselstrom-Magnetfeld erzeugt, um eine Position innerhalb des Kopfes und Halses während der IGS anzuzeigen, und der Fremdstoffe innerhalb des Kopfes und Halses während der IGS durch eine in der Mitte ausgebildete Saugöffnung absaugt; und
einen EM-Endoskopschaft (400), der mit einer Seite in den Kopf und Hals des Patienten bei IGS eingeführt ist, während die andere Seite mit einem handelsüblichen Endoskop verbunden ist, und der als Reaktion auf das Wechselstrom-Magnetfeld den induzierten Strom erzeugt, um während der IGS eine Position des handelsüblichen Endoskops innerhalb des Kopfes und Halses anzuzeigen.

## Revendications

1. Un appareil de suivi de position pour la chirurgie guidée par image de la tête et du cou (IGS) (100) utilisant un champ magnétique, comprenant :
une partie principale du corps (110) ;
un générateur de champ (120) qui est connecté à la partie principale du corps (110) et génère extérieurement un champ magnétique alternatif (AC) autour d'une zone affectée via une bobine interne selon un signal de contrôle transmis par la partie principale du corps (110) ;
un traceur électromagnétique (EM) de patient (130) dont un côté est porté sur la tête et le cou du patient dans l'IGS tandis que l'autre côté est relié à la partie principale du corps (110) et détecte le champ magnétique alternatif et transmet un signal de résultat de détection à la partie principale du corps (110) ;
un pointeur EM (140) qui a un côté inséré dans la tête et le cou du patient dans l'IGS tandis que l'autre côté est relié à la partie principale du corps (110) et génère un courant induit en réponse au champ magnétique alternatif pour indiquer une position dans la zone affectée ;
un chariot de déménagement (150) qui porte la partie principale du corps (110) et le générateur de champ (120) montés dessus, configuré pour être mobile ;
un traceur d'instruments EM (160) configuré de sorte qu'un côté soit relié à la partie principale du corps (110) et l'autre côté à un instrument chirurgical commercial, qui guide l'instrument chirurgical commercial inséré dans la tête et le cou du patient dans l'IGS pour générer le courant induit en réponse au champ magnétique alternatif ;
une pince à pinces (170) fixée au chariot en mouvement (150) et dont un côté est conçu pour fixer les pinces chirurgicales ;
une serre polyvalente (180) fixée au chariot de mouvement (150) qui serre et fixe l'instrument chirurgical commercial ; et
un chariot de stockage mobile (190) qui dispose d'un espace de stockage pour recevoir le traceur patient EM (130), le pointeur EM (140), le traceur d'instruments EM (160), la pince pince à pinces (170) et la pince polyvalente (180), et configuré pour être mobile,
où, comme le pointeur EM (140) génère le courant induit en réponse au champ magnétique AC généré à l'extérieur par le générateur de champ (120), lorsque le traceur EM patient (130) porté sur la tête et le cou du patient dans l'IGS transmet le signal de détection détectant le courant induit, la partie principale du corps (110) calcule une distance, position, et direction du pointeur EM (140) dans la tête et le cou du patient dans l'IGS et mappe une image générée en calculant la distance, la position et la direction du pointeur EM (140) à une image radiographique faciale précédemment capturée du patient, et l'image mappée est envoyée via un dispositif d'affichage relié à la partie principale du corps (110), **caractérisé par** un trou de support fixe (121) est formé de chaque côté et à la surface arrière du générateur de champ (120), avec lequel un support fixe pour le montage sur le chariot mobile (150) ou un lit chirurgical est fixé ou fixé, et le générateur de champ (120) reçoit l'énergie pour générer le champ magnétique alternatif à partir de la partie principale du corps (110).

2. L'appareil de suivi de position selon la revendication 1, où une partie avant de la partie principale du corps (110) est équipée de :
un interrupteur d'alimentation (111) ;
au moins deux ports de connexion USB 3.0 (112) dans lesquels un périphérique mémoire USB est insérable ou inséré ;
un port de connexion (113) pour un générateur de champ dans lequel une borne de câble du générateur de champ (120) est insérable ou insérée ;
un port de connexion (114) pour un suivi EM patient dans lequel un terminal câble du suivi EM patient (130) est insérable ou inséré ;
un port de connexion (115) pour un pointeur EM dans lequel une borne de câble du pointeur EM (140) est insérable ou insérée ; et
un port de connexion (116) pour un pointeur de tube EM dans lequel une borne de câble du pointeur de tube EM est insérable ou insérée, et
Une partie arrière de la partie principale du corps (110) est fournie de :
un réseau électrique (117) ;
un terminal d'égalisation du potentiel de mise à la terre (118) ; et
un port de connexion supplémentaire (119) comprenant un port USB 2.0, un port DVI et un port HDMI.

3. L'appareil de suivi de position, selon l'une des revendications 1 ou 2, où la partie principale du corps (110) amplifie le signal de résultat de détection transmis par le suivi patient EM (130) à l'aide d'un module d'amplification de l'unité d'interface d'étage intermédiaire (ISIU), pré-traite, dans un SCU, le signal de résultat de détection amplifié par le module d'amplification ISIU, puis traite le signal de résultat de détection prétraité en informations de position tridimensionnelles, et transmet le signal traité à un module de sortie vidéo à envoyer via le dispositif d'affichage.

4. L'appareil de suivi de position selon l'une des revendications 2 à 3, dans lequel lorsque l'interrupteur d'alimentation (111) est actionné en état marché par un praticien médical, la partie principale du corps (110) exécute une application dédiée pour le suivi de position de l'IGS, récupère les données d'image du patient correspondant à partir d'informations d'identification uniques pour chaque patient, puis envoie une image DICOM vers l'appareil d'affichage.

5. L'appareil de suivi de position, selon l'une des revendications 1 à 4, où la partie principale du corps (110) récupère les données d'image du patient correspondant, puis envoie une notification au praticien médical via l'affichage pour vérifier à nouveau si les informations d'identification uniques et celles du patient à opérer correspondent.

6. L'appareil de suivi de position selon l'une des revendications 1 à 5, où un tampon adhésif médical est placé sur une surface inférieure du traceur patient EM (130), et le traceur patient EM (130) est fixé au front du patient grâce à la résistance adhésive du tampon adhésif médical.

7. L'appareil de suivi de position selon la revendication 6, dans lequel lorsque le traceur EM patient (130) est fixé au front du patient via le tampon adhésif médical, la partie principale du corps (110) affiche un écran pour sélectionner un type de repère ou un type de surface via l'affichage pour mesurer une valeur d'enregistrement.

8. L'appareil de suivi de position selon la revendication 7, dans lequel lorsque le type de repère est sélectionné par le médecin, la partie principale du corps (110) affiche un écran guide pour désigner les points de référence sur l'écran, et guide le praticien médical pour déplacer la position du pointeur EM (140) à travers l'écran guide afin que les positions désignées soient en contact séquentiel.

9. L'appareil de suivi de position, selon l'une des revendications 7 à 8, dans lequel, lorsque le type de surface est sélectionné par le médecin, la partie principale du corps (110) affiche un écran guide pour désigner les points de départ sur l'écran, et guide le médecin pour déplacer la position du pointeur EM (140) à travers l'écran de guidage afin que le pointeur EM (140) entre en contact avec la surface du visage du patient le long d'un Un chemin prédéfini commençant par le point de départ.

10. L'appareil de suivi de position selon l'une des revendications 1 à 9, où la partie principale du corps (110) guide un processus de guidage du mouvement de position du pointeur EM (140) à répéter jusqu'à ce que la valeur d'enregistrement atteigne une valeur d'enregistrement prédéfinie.

11. L'appareil de suivi de position, selon la revendication 10, dans lequel, dans le processus de guidage du praticien médical pour déplacer la position du pointeur EM (140) à travers l'écran de guidage afin que le pointeur EM (140) soit contacté le long du chemin prédéfini à partir du point de départ sur la surface du visage du patient, la partie principale du corps (110) désigne une zone entre le front et les sourcils du patient comme point de départ, puis guide un premier chemin qui relie la zone de l'œil gauche à celle droite du patient en forme de huit, un second chemin qui traverse le nez du patient et mène à un philtrum, et un troisième chemin qui commence au philtrum et est élevé jusqu'à une glabelle tout en zigzaguant sur un pont du nez à travers l'écran guide.

12. L'appareil de suivi de position, selon l'une des revendications 10 à 11, dans lequel lorsque le pointeur EM (140) est positionné dans une plage effective du champ magnétique AC généré à l'extérieur via le générateur de champ (120) dans la tête et le cou du patient dans l'IGS, la partie principale du corps (110) affiche une icône verte à l'écran du dispositif d'affichage, et lorsque le pointeur EM (140) est déterminé hors de la portée effective du champ magnétique AC dans la tête et le cou du patient dans l'IGS, la partie principale du corps (110) affiche une icône rouge et affiche un écran d'alerte pour signaler que le pointeur EM (140) est hors de portée effective sur l'écran.

13. L'appareil de suivi de position selon l'une des revendications 1 à 12, où la pince polyvalente (180) comprend :
une petite pince tout autour (181) qui serre l'instrument chirurgical commercial d'un diamètre de 2 mm à 6 mm et fixe l'instrument chirurgical commercial au chariot mobile (150) ;
une pince moyenne polyvalente (182) qui serre l'instrument chirurgical commercial d'un diamètre de 6 mm à 10 mm et fixe l'instrument chirurgical commercial sur le chariot en mouvement (150) ; et
une grande pince polyvalente (183) qui fixe l'instrument chirurgical commercial d'un diamètre de 10 mm à 16 mm et fixe l'instrument chirurgical commercial au chariot en mouvement (150).

14. L'appareil de suivi de position selon l'une des revendications 1 à 13, comprenant en outre :
un suivi d'instruments EM supplémentaire (200) configuré de manière à ce qu'un côté soit relié à la partie principale du corps (110) et l'autre côté à un cathéter ballonnet commercial, qui guide le cathéter ballonnet commercial inséré dans la tête et le cou du patient dans l'IGS pour générer le courant induit en réponse au champ magnétique alternatif ;
un pointeur de tube EM (300) dont un côté est inséré dans la tête et le cou du patient dans l'IGS tandis que l'autre côté est relié à la partie principale du corps (110), génère le courant induit en réponse au champ magnétique alternatif pour indiquer une position dans la tête et le cou pendant l'IGS, et aspire les substances étrangères à l'intérieur de la tête et du cou lors de l'IGS à travers un trou d'aspiration formé au centre ; et
un arbre d'endoscope EM (400) qui insère un côté dans la tête et le cou du patient dans l'IGS tandis que l'autre côté est couplé à un endoscope commercial, et génère le courant induit en réponse au champ magnétique alternatif pour indiquer la position de l'endoscope commercial dans la tête et le cou lors de l'IGS.
